(19) Europäisches Patentamt
European Patent Office
Office européen des brevets

(11) Veröffentlichungsnummer: **0 250 436**
**B1**

(12) # EUROPÄISCHE PATENTSCHRIFT

(45) Veröffentlichungstag der Patentschrift: **15.11.89**

(51) Int. Cl.⁴: **C 07 D 213/89**

(21) Anmeldenummer: **86902402.6**

(22) Anmeldetag: **10.04.86**

(86) Internationale Anmeldenummer: **PCT/EP 86/00211**

(87) Internationale Veröffentlichungsnummer: **WO 86/06067 (23.10.86 Gazette 86/23)**

(54) **MEHRFACH SUBSTITUIERTE PYRIDIN-1-OXIDE, VERFAHREN ZU IHRER HERSTELLUNG, DIE SIE ENTHALTENDEN ARZNEIMITTEL UND IHRE VERWENDUNG.**

(30) Priorität: **18.04.85 DE 3514073**

(43) Veröffentlichungstag der Anmeldung: **07.01.88 Patentblatt 88/1**

(45) Bekanntmachung des Hinweises auf die Patenterteilung: **15.11.89 Patentblatt 89/46**

(84) Benannte Vertragsstaaten: **AT BE CH DE FR GB IT LI LU NL SE**

(56) Entgegenhaltungen:
**DE-A-2 900 504**
**US-A-4 115 396**

(73) Patentinhaber: **HOECHST AKTIENGESELLSCHAFT, Postfach 80 03 20, D-6230 Frankfurt am Main 80 (DE)**

(72) Erfinder: **GREVE, Wilfried, Wingertstrasse 43b, D-6074 Rödermark (DE)**
Erfinder: **ELBEN, Ulrich, Eschenstrasse 23, D-6200 Wiesbaden (DE)**
Erfinder: **RUDOLPHI, Karl, Hornauer Strasse 162, D-6233 Kelkheim (Taunus) (DE)**
Erfinder: **SCHINDLER, Ursula, Reinhardswaldweg 1, D-6082 Mörfelden- Walldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

EP 0 250 436 B1

**Beschreibung**

In der DE-OS-2 900 504 ist die Herstellung bronchospasmolytisch wirksamer substituierter 3-Nitro- (bzw. Cyano-)4-aminopyridine beschrieben. Die entsprechenden Pyridin-1-oxide werden in dieser Druckschrift aber nicht beschrieben. Gegenstand der US-PS-3 547 935 sind herbizid wirksame 3-Nitropyridine, wobei die Substituentendefinition zur Beschreibung der allgemeinen Strukturformel unter anderem auch 2,6-Dialkyl-4-dialkylamino-3-nitropyridin-1-oxide umfaßt, ohne daß aber derartige Verbindungen anhand von Beispielen oder durch Nennung offenbart werden. Weiterhin beschreiben die beiden DE-OS-3 209 274 und 3 209 276 3-Pyridincarbonsäureester und 3,5-Pyridindicarbonsäureester mit einem über ein C-Atom gebundenen Substituenten in 4-Stellung, die sich zur Therapie ischämie- und/oder hypoxiebedingter Erkrankungen eignen sollen. Aus der Literatur sind ferner das 3-Phenoxypyridin und Derivate (J. Med. Chem 24, 346 (1981) und BE-PS-876 389) und 2-Cyan-3-phenoxypyridin-1-oxide (US-PS-4 187 379 und 4 229 457) bekannt, denen antiamnestische Eigenschaften zugeschrieben werden.

Überraschend wurde nun gefunden, daß durch die Einführung eines Sauerstoffatoms in die 1-Position von Pyridinderivaten des in der DE-OS-2 900 504 beschriebenen Strukturtyps neue Verbindungen erhalten werden, die keine bronchospasmolytische Aktivität mehr zeigen, sondern andere wertvolle pharmakologische Eigenschaften besitzen. Im Vordergrund steht hierbei eine ausgeprägte gehirnprotektive Wirkung, die von thrombocytenaggregationshemmenden und antiödematösen Effekten begleitet wird. Die erfindungsgemäßen Verbindungen sind darin den Verbindungen der übrigen zum Stand der Technik genannten Literaturzitate, von denen ebenfalls repräsentative Vertreter in die pharmakologische Untersuchung miteinbezogen worden sind, erheblich überlegen, denn die bekannten Verbindungen haben sich als wesentlich schwächer aktiv oder gar unwirksam erwiesen. Darüber hinaus eignen sich die erfindungsgemäßen Verbindungen auch als Ausgangsstoffe für die Herstellung weiterer wertvoller Pharmaka.

Die vorliegende Erfindung betrifft folglich neue, mehrfach substituierte Pyridin-1-oxide einschließlich der zugehörigen Salze, Verfahren zu ihrer Herstellung, sie enthaltende Arzneimittel, insbesondere solche, die die prophylaktische und kurative Behandlung von vaskulär und degenerativ bedingten Gehirnerkrankungen gestatten, und die Verwendung der Pyridin-1-oxide der Formel I und/oder der Salze zur Herstellung von Arzneimitteln, die zur Vorbeugung und Behandlung von vaskulär und degenerativ bedingten Gehirnerkrankungen bestimmt sind.

Gegenstand sind somit die substituierten Pyridin-1-oxide der Formel I (s. Anspruch 1), in der

$R^1$ und $R^2$ jeweils gleich oder verschieden sind und für geradkettiges oder verzweigtes Alkyl mit 1 bis 6, vorzugsweise mit 1 bis 3 C-Atomen, insbesondere Methyl stehen,

$R^3$ Wasserstoff und

$R^4$ Mercaptoalkyl mit bis zu 4 C-Atomen bedeuten oder

$R^3$ und $R^4$ zusammen mit dem 4-ständigen N-Atom einen unsubstituierten oder bis zu zwei gleiche oder verschiedene Substituenten aus der Gruppe Hydroxy und Alkyl mit bis zu 2 C-Atomen tragenden fünf- bis siebengliedrigen gesättigten heterocyclischen Ring mit bis zu 2 Heteroatomen bilden, wobei das zweite Heteroatom Sauerstoff, Schwefel, der bis zu zwei Sauerstoffatome tragen kann, oder Stickstoff in Form der $NR^6$-Gruppe darstellt, worin $R^6$ Wasserstoff, Alkyl mit bis zu 2 C-Atomen, Phenylalkyl mit bis zu 2 C-Atomen im Alkylteil oder Phenyl ist und die Phenylringe der beiden letztgenannten Reste bis zu zwei gleiche oder verschiedene Substituenten aus der Gruppe Halogen und Methoxy tragen können, und

X die Cyan- oder Nitrogruppe oder die Gruppe -CO-$R^5$ bedeutet, in der $R^5$ für die Amino-, Hydroxy- oder eine Alkoxygruppe mit 1 bis 4 C-Atomen steht,

und die physiologisch verträglichen Salze dieser Verbindungen.

$R^1$ und $R^2$ enthalten zusammen vorzugsweise nicht mehr als 8 und insbesondere nicht mehr als 6 C-Atome. In $R^4$ enthält der Mercaptoalkylrest vorzugsweise bis zu 2 C-Atome. In dem aus $R^3$ und $R^4$ gebildeten Ring und im Phenylalkylrest kann der Alkylrest ein oder zwei C-Atome enthalten und das Halogen am Phenylring insbesondere Fluor, Chlor oder Brom sein.

Bevorzugt sind solche Verbindungen der Formel I und deren Salze, bei denen $R^1$ und $R^2$ jeweils für 1 bis 3 C-Atome, vorzugsweise Methyl stehen, die Cyan- oder Nitrogruppe bedeutet und die Gruppierung $NR^3R^4$ einen unsubstituierten oder mit einem Alkyl mit bis zu 2 C-Atomen substituierten heterocyclischen Ring mit mindestens 4 C-Atomen, vorzugsweise einen Thiomorpholin-, Morpholin-, Pyrrolidin-, Piperidin-, Hexamethylenimin-, Piperazin- oder Homopiperazin-Ring oder einen unsubstituierten oder im Phenylkern mit einem Halogen substituierten 4-Phenylpiperazinrest darstellt.

Unter diesen Verbindungen sind wiederum jene der Formel I und deren Salze besonders hervorzuheben, in denen $R^1$ und $R^2$ jeweils Methyl, X die Cyangruppe und $NR^3R^4$ den Thiomorpholin-, Piperidin- oder Hexamethylenimin-Ring bedeuten, und vor allem das 3-Cyan-2,6-dimethyl-4-(4-thiomorpholinyl)-pyridin-1-oxid.

Die Verbindungen der Formel I sind neu und besitzen, wie eingangs erwähnt, wertvolle pharmakologische, vor allem gehirnprotektive Eigenschaften.

Gegenstand der Erfindung ist weiterhin ein Verfahren zur Herstellung der Pyridin-1-oxide gemäß Formel I und ihrer physiologisch verträglichen Salze, das dadurch gekennzeichnet ist, daß man

a) eine Verbindung der Formel II zu einem Pyridin-1-oxid der Formel III oxydiert und dieses anschließend mit einem Amin der Formel $HNR^3R^4$ (IV) zu einem Pyridin-1-oxid der Formel (I) umsetzt, wobei $R^1$ bis $R^4$ und X die oben genannten Bedeutungen haben und Z ein Halogenatom darstellt, insbesondere Chlor oder Brom, oder

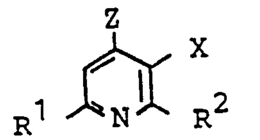

(II)

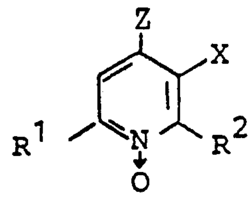

(III)

b) eine Verbindung der Formel V in der $R^1$ bis $R^4$ und X die vorgenannten Bedeutungen haben, zu einem Pyridin-1-oxid der Formel I oxydiert, wobei eine etwa in dem Rest $-NR^3R^4$ enthaltene Thioätherfunktion gleichzeitig mitoxydiert wird,

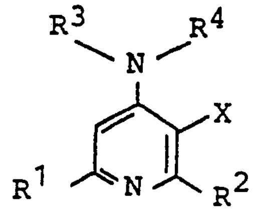

(V)

und das gemäß a) oder b) erhaltene Produkt isoliert oder

c) zur Herstellung einer Verbindung der Formel I, in der die $NR^3R^4$-Gruppe eine Sulfoxy- oder Sulfongruppe enthält, eine nach dem Verfahren a) erhaltene Thioätherverbindung nachträglich, gegebenenfalls schrittweise am Schwefel oxydiert oder

d) zur Herstellung einer Verbindung der Formel I, in der X für den Rest $-CONH_2$ steht, eine 3-Cyanverbindung der Formel I zum Amid hydrolysiert oder einen 3-Carbonsäureester der Formel I mit Ammoniak umsetzt oder

e) zur Herstellung einer Verbindung der Formel I, in der X die Carboxylgruppe bedeutet, einen 3-Carbonsäureester der Formel I hydrolysiert,

wobei man die Verbindungen der Formel I entweder in freier Form isoliert oder mit geeigneten, d. i. physiologisch verträglichen, Säuren oder, für den Fall, daß X eine COOH-Gruppe darstellt, auch mit geeigneten, d. i. physiologisch verträglichen Basen physiologisch verträgliche Salze bildet.

Für die Herstellung von Säureadditionssalzen kommen beispielsweise Mineralsäuren wie Schwefel- oder Phosphorsäure oder Halogenwasserstoffsäuren, insbesondere Salzsäure, und organische Säuren wie ein- bis dreibasische Carbonsäuren, z. B. Essig-, Milch-, Malein-, Fumar-, Oxal-, Wein-, Zitronen- oder Glukonsäure, oder andere physiologisch verträgliche Säuren, wie Sulfonsäuren, z. B. p-Toluolsulfon, Methansulfon-, Trifluormethylsulfon- und Cyclohexylamidosulfonsäure in Frage.

Die Verbindungen der Formel I, in denen X eine Carboxylgruppe darstellt, können auch mit basischen Reagentien, wie Hydroxiden, Alkoholaten, Carbonaten und Hydrogencarbonaten stabile, wasserlösliche Alkali- und Erdalkalisalze bilden.

Die Ausgangsstoffe der Formeln II und V sind zumeist literaturbekannt oder aber nach in der Literatur beschriebenen Methoden leicht herstellbar.

Als geeignete Verbindungen II seien beispielsweise genannt die symmetrischen 2,6-Dialkyl-4-halogen-3-nitropyridine, wie 4-Chlor-2,6-dimethyl-3-nitropyridin (P. Nantka-Namirski, Acta Polon. Pharm. 18, 449 (1961) und 4-Chlor-2,6-dipropyl-3-nitropyridin, die sich aus Acylessigsäureestern über die 6-Alkyl-3-acyl-2,3-dihydro-2,4-dioxo-pyrane durch Umsetzung mit Ammoniak, anschließende Nitrierung in 3-Stellung und Halogenierung der 4-Position gewinnen lassen; ferner eignen sich 2,6-Dialkyl-3-cyan-4-halogenpyridine, wie 4-Chlor-3-cyan-2,6-dimethylpyridin (T. Kato et al., Yakugaku Zasshi 91, 740 (1971)) und 4-Chlor-3-cyan-2,6-dipropylpyridin, und 2,6-Dialkyl-4-halogen-pyridin-3-carbonsäureester, wie 2,6-Dimethyl-4-chlorpyridin-3-carbonsäureäthylester, die durch Kondensation von 3-Alkyl-3-aminoacrylsäureestern mit Phosphoroxytrichlorid (J. N. Phillips et al., Angew. Chem. 88, 539 (1976)) oder mit Acylessigsäureestern (Tschechoslow. Patentschrift 147 252; CA. 78, 159 448 j) herstellbar sind, sowie deren unsymmetrische 2,6-Dialkylderivate, wie die 3-substituierten 2-Hexyl-6-methyl- bzw. 6-Hexyl-2-methyl-4-chlorpyridine, die man beispielsweise durch Umsetzung entsprechender 3-Alkyl-3-aminoacrylsäureester mit Diketen (T. Kato et al., Yakugaku Zasshi 91, 740 (1971) oder beliebigen Acylessigsäureestern und anschließende Verseifung, Decarboxylierung, Nitrierung, Isomerentrennung und Halogenierung aufbauen kann. Darüber hinaus sind die Verbindungen der Formel II, in denen X für die Cyangruppe steht, auch aus den entsprechenden Nitroverbindungen durch Reduktion der Nitrogruppe zur

Aminofunktion, deren Diazotierung und nachfolgenden nucleophilen Austausch gegen die Cyangruppe (C. Räth, Liebigs Ann. Chem. 486, 95 (1931)), vorteilhaft unter Kupfer (I)-salz-Katalyse, zugänglich. Diese Reaktionsfolge ist auf die 3-Nitropyridin-1-oxide der Formel III übertragbar und gestattet somit auch hier deren Umwandlung in die entsprechenden 3-Cyanpyridin-1-oxide.

Die Ausgangsverbindungen V sind größtenteils aus der DE-OS-2 900 504 bekannt oder aber auf analoge Weise durch Umsetzung der 3-substituierten 2,6-Dialkyl-4-halogenpyridine II mit den Aminen IV leicht herstellbar.

Für die N-Oxydation sowohl der Pyridinderivate II zu den Zwischenprodukten III entsprechend Verfahren a) als auch der substituierten 4-Aminopyridine V zu den erfindungsgemäßen Verbindungen I entsprechend Verfahren b) eignen sich z. B. Wasserstoffperoxyd, Perborate, vorzugsweise aber organische Percarbonsäuren, wie Perameisen-, Peressig-, Trifluorperessig-, Monopermalein-, Monoperbernstein-, Perbenzoe-, 4-Nitroperbenzoe-, Monoperphthal- und insbesondere 3-Chlorperbenzoesäure. Jedoch ist auch eine elektrochemische Oxydation möglich.

Die Umsetzung mit den Percarbonsäuren wird zweckmäßig in einem gegenüber den Reaktionsteilnehmern inerten Lösungs- oder Verteilungsmittel durchgeführt, das erfahrungsgemäß einen beachtlichen Einfluß auf die Reaktionsgeschwindigkeit ausübt. Im allgemeinen führt man die Reaktion bei Atmosphärendruck aus, wenngleich die Anwendung von Über- bzw. Unterdruck ebenfalls möglich ist. Da Lösungs- bzw. Verteilungsmittel, die mit den Percarbonsäuren Wasserstoffbrückenbindungen bilden können, im allgemeinen die Reaktionsgeschwindigkeit herabsetzen, sind aromatische Kohlenwasserstoffe, wie Benzol, Toluol oder Xylole, und halogenierte Kohlenwasserstoffe, wie Dichlormethan, Chloroform oder Tetrachlormethan, bzw. Gemische derselben, gegenüber Äthern, wie Diäthyläther, Dioxan, Tetrahydrofuran oder Äthylenglykoldimethyläther, Alkoholen, Estern und Carbonsäuren häufig bevorzugt. Die Umsetzung erfolgt gewöhnlich bei Temperaturen zwischen +10°C und der Siedetemperatur des jeweiligen Lösungsmittel, d. i. des Reaktionsmediums, vorzugsweise bei Raumtemperatur oder 20 bis 70°C, wobei die Reaktionszeit bis zu mehreren Stunden reichen kann. Gewöhnlich setzt man Percarbonsäuren in isolierter Form zur Reaktion ein, sie können aber auch, beispielsweise aus der entsprechenden Carbonsäure und Wasserstoffperoxyd, im Reaktionsansatz in situ erzeugt werden.

Auch bei der Umsetzung der in 4-Stellung halogenierten Pyridin-1-oxide III mit den Aminen IV entsprechend Verfahren a) wird zweckmäßig in einem gegenüber den Reaktionspartnern inerten Lösungs- oder Verteilungsmittel gearbeitet. Hierfür kommen z. B. Alkohole, wie Methanol, Äthanol, Isopropanol, n-Propanol, die verschiedenen Butanole, sowie Gemische derselben, oder auch deren Mischungen mit Äthern, wie Tetrahydrofuran und Dioxan, oder Kohlenwasserstoffen, wie Benzol, Toluol und Xylol, sowie aprotische Lösungsmittel, wie Pyridin, Dimethylformamid, Dimethylacetamid, Dimethylsulfoxyd und Hexamethylphosphorsäuretriamid in Frage.

Bei der Umsetzung der Verbindungen III mit den Aminen IV arbeitet man vorteilhaft mit der mindestens zweifach molaren Menge des Amins; auch der Einsatz äquimolarer Mengen beider Reaktionspartner ist möglich, doch empfiehlt sich dann die Zugabe eines säurebindenden Mittels, z. B. eines Alkali- oder Erdalkalihydroxids oder -carbonats oder auch einer organischen Base, wie Triäthylamin, in mindestens stöchiometrischer Menge. Die Reaktion wird im allgemeinen bei Temperaturen zwischen 0°C und dem Siedepunkt des jeweiligen Lösungsmittels, d. i. des Reaktionsmediums, vorzugsweise zwischen 20 und 100°C durchgeführt, wobei die Reaktionszeit bis zu mehreren Stunden betragen kann.

Geeignete Amine IV sind beispielsweise Pyrrolidin, Piperidin, 4-Hydroxypiperidin, Hexamethylenimin, Morpholin, Thiomorpholin, 2-Methylthiomorpholin, 2-Mercaptoäthylamin, Piperazin, Homopiperazin und die monosubstituierten Piperazine, wie 1-Methyl-, 1-Benzyl-, 1-Phenyl-, 1-(4-Methoxyphenyl)-, 1-(3-Chlorphenyl)- oder 1-(4-Fluorphenyl)-piperazin sowie Thiomorpholin-1-oxid und -1,1-dioxid.

Die Oxydation von erfindungsgemäßen Verbindungen der Formel I mit einem Thioäthergruppen enthaltenden $NR^3R^4$-Rest zu Sulfoxiden bzw. Sulfonen gemäß Verfahren c) gelingt nach üblichen Methoden, beispielsweise zur Herstellung von Sulfoxiden mit Salpetersäure, elementarem Chlor, Perboraten, den obengenannten Percarbonsäuren und Wasserstoffperoxyd, vorzugsweise aber mit Perjodaten, wie Tetrabutylammoniumperjodat und Natriummetaperjodat. Zur Herstellung von Sulfonen eignen sich im Prinzip die gleichen Oxydationsmittel, wobei jedoch Salpetersäure, Chlor und insbesondere Wasserstoffperoxyd bevorzugt sind. Dabei dient bevorzugt Wasser als Verdünnungsmittel. Häufig bewährt sich hierbei auch der Zusatz einer Carbonsäure, wie Essigsäure. Die Reaktionstemperaturen liegen in der Regel zwischen -20° und +100°C, vorzugsweise zwischen 0° und 80°C.

Die etwaige Umwandlung der erfindungsgemäßen Cyanverbindungen und Carbonsäureester der Formel I in die zugehörigen Amide der Formel I gemäß Verfahren d) sowie der erfindungsgemäßen Carbonsäureester in die Carbonsäuren der Formel I entsprechend Verfahren e) erfolgt in üblicher Weise. So kann die Hydrolyse der Cyanverbindungen zu den Amiden unter sauren oder bevorzugt alkalischen, insbesondere stark alkalischen Bedingungen durchgeführt werden, wobei man vornehmlich in niederen Alkoholen, Äthern oder insbesondere Wasser unter Zusatz alkalischer Reagentien, vorzugsweise Alkalihydroxiden, wie Natrium- oder Kaliumhydroxid bei Temperaturen zwischen 20° und dem Siedepunkt des jeweiligen Lösungsmittels arbeitet. Auch die Aminolyse der erfindungsgemäßen Carbonsäureester mit Ammoniak zu den Amiden der Formel I wird zweckmäßig in einem gegenüber den Reaktionspartnern inerten Lösungsmittel, vorzugsweise einem Alkohol, wie Methanol, Äthanol oder Isopropanol, vorgenommen, wobei sich die Anwendung höherer Drucke und Temperaturen zwischen 100 und 200°C empfiehlt. Bei der Hydrolyse der erfindungsgemäßen Carbonsäureester

zu den Säuren der Formel I kann ebenfalls sowohl unter sauren als auch alkalischen Bedingungen gearbeitet werden. Die alkalische Hydrolyse ist aber bevorzugt und wird vorteilhaft in einem Lösungsmittel, wie Wasser, einem Äther, Keton oder niederen Alkohol, vorzugsweise Äthylenglykol, in Gegenwart von alkalischen Reagentien, vorzugsweise Alkalihydroxiden oder -carbonaten, insbesondere Natrium- oder Kaliumhydroxid, gegebenenfalls bei erhöhten Temperaturen durchgeführt.

Die erfindungsgemäßen Pyridin-1-oxide der Formel I und ihre physiologisch verträglichen Salze können aufgrund ihrer pharmakologischen Eigenschaften als Arzneimittel, insbesondere als solche zur prophylaktischen und kurativen Behandlung von vaskulär und degenerativ bedingten Erkrankungen des Gehirns Verwendung finden, wobei man sie entweder allein, z. B. in Form von Mikrokapseln, in Mischungen untereinander oder in Kombination mit geeigneten Hilfs- und/oder Trägerstoffen verabreicht.

Gegenstand der Erfindung sind somit auch Arzneimittel, die mindestens eine Verbindung der Formel I, gegebenenfalls in Form eines ihrer physiologisch verträglichen Salze, als Wirkstoff enthalten oder daraus bestehen, und eine echte Bereicherung der Pharmazie darstellen.

Die erfindungsgemäßen Arzneimittel werden im allgemeinen oral oder parenteral verabreicht; aber auch eine rektale oder perkutane Anwendung ist prinzipiell möglich. Geeignete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Aerosole, Tropfen oder injizierbare Lösungen in Ampullenform, wobei auch die Trockenampulle als eine spezielle Zubereitungsform miteingeschlossen ist, sowie Präparate mit protrahierter Wirkstoff-Freigabe, bei deren Herstellung gewöhnlich Hilfsmittel, wie Trägerstoffe, Spreng-, Binde-, Überzugs-, Quellungs-, Gleit- oder Schmiermittel, Geschmackstoffe, Süßungsmittel, Puffersubstanzen, Antioxidantien oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyäthylenglykole und Lösungsmittel, wie etwa steriles Wasser, Alkohole, Glycerin und andere mehrwertige Alkohole genannt.

Vorzugsweise werden die pharmazeutischen Präparate in Dosierungseinheiten hergestellt und verabreicht, wobei jede Einheit als aktiven Bestandteil eine bestimmte Dosis einer Verbindung gemäß Formel I, gegebenenfalls in Form eines ihrer physiologisch verträglichen Salze enthält. Bei festen Dosierungseinheiten, wie Tabletten, Kapseln und Suppositorien, kann diese Dosis bis zu 1000 mg bevorzugt jedoch 100 bis 300 mg, und bei Injektionslösungen in Ampullenform bis zu 200 mg, vorzugsweise aber 20 bis 100 mg, betragen.

Für die Behandlung eines erwachsenen Patienten sind - je nach Wirksamkeit der Verbindungen gemäß Formel I und ihrer Salze am Menschen - Tagesdosen von 100 bis 2000 mg Wirkstoff, vorzugsweise 300 bis 900 mg, bei oraler Verabreichung und von 5 bis 500 mg, bevorzugt 20 bis 200 mg, bei intravenöser Applikation indiziert. Unter Umständen können jedoch auch höhere oder niedrigere Tagesdosen empfehlenswert sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmen Intervallen erfolgen.

Schließlich können die Pyridin-1-oxide der Formel I sowie deren Salze bei der Herstellung der vorgenannten galenischen Zubereitungsformen auch zusammen mit anderen geeigneten Wirkstoffen, beispielsweise Antihypertensiva einschließlich β-Rezeptoren- und Calciumkanal-Blockern, antianginösen und positiv inotrop wirkenden Mitteln, Diuretika, Sedativa, Antidepressiva, Antihyperlipidämika, Antithrombotika und Vasotherapeutika, formuliert werden.

**Beispiele:**

Die Struktur der nachstehend beschriebenen Verbindungen wurde durch Elementaranalyse sowie IR- und $^1$H-NMR-Spektren bewiesen.

1) 3-Cyan-2,6-dimethyl-4-(4-thiomorpholinyl)-pyridin-1-oxid-hydrochlorid:

a) Man löste 50 g (0,3 Mol) 4-Chlor-3-cyan-2,6-dimethylpyridin in 1 l Dichlormethan, gab portionsweise bei Raumtemperatur 130 g (0,6 Mol) 80 %-ige m-Chlorperoxybenzoesäure hinzu, rührte über Nacht weiter und filtrierte die ausgefallene m-Chlorbenzoesäure ab. Das Filtrat wurde mit Wasser versetzt, mit festem Kaliumhydroxid auf pH 12 eingestellt und die beiden Phasen gegeneinander ausgeschüttelt. Die Dichlormethanphase wurde abgetrennt, über Natriumsulfat getrocknet, filtriert und eingeengt. Man erhielt das 4-Chlor-3-cyan-2,6-dimethylpyridin-1-oxid als feste Verbindung, die bei 140°C sinterte. Ausbeute: 49,3 g (90 % der Theorie)

b) Man löste 49,3 g (0,27 Mol) der vorstehend erhaltenen Verbindung, 31,0 g (0,30 Mol) Thiomorpholin und 40 ml (0,28 Mol) Triäthylamin in 150 ml Methanol und erhitzte 10 Stunden unter Stickstoff zum Rückfluß. Nach beendeter Reaktion wurde das Lösungsmittel unter vermindertem Druck abdestilliert, der Rückstand in 200 ml Dichlormethan aufgenommen und 100 ml Wasser zugefügt. Man säuerte mit 2 N Salzsäure bis pH 5 an. Die Wasserphase wurde abgetrennt, die verbleibende Dichlormethanphase über Natriumsulfat getrocknet, filtriert und unter vermindertem Druck zur Trockne eingeengt. Der Rückstand wurde aus Isopropanol/Wasser (Volumenverhältnis 3 : 1) umkristallisiert.

Die so gereinigte Base wurde in der Hitze mit möglichst wenig Äthanol gelöst und 8 N äthanolische HCl zugegeben. Man engte die Lösung bis zur Trübung ein und ließ im Kühlschrank auskristallisieren. Ausbeute:

46,3 g (60 % der Theorie)
$C_{12}H_{16}ClN_3SO$ (MG: 285, 79); Schmelzpunkt 237 - 239° C
Analyse:

Ber.: C 50,43 % H 5,64 % Cl 12,40 % N 14,70 % S 11,22 %
Gef.: C 50,49 % H 5,72 % Cl 12,37 % N 14,70 % S 11,20 %

2) 3-Cyan-2,6-dimethyl-4-(4-thiomorpholinyl-S-oxid)-pyridin-1-oxid:
9 g (0,03 Mol) der Verbindung gemäß Beispiel 1b wurden in Wasser gelöst und bei 0° C mit 7,07 g (0,033 Mol) Natriumperjodat versetzt. Man ließ das Reaktionsgemisch auf Raumtemperatur kommen, rührte 20 Stunden nach, verdünnte den Ansatz mit Methanol und filtrierte vom ausgefallenen Niederschlag ab. Die wäßrige Phase wurde gegen Dichlormethan ausgeschüttelt, dann mit Natriumhydrogencarbonat neutralisiert und nochmals mit Dichlormethan ausgeschüttelt. Diese Dichlormethanphase wurde eingeengt und der feste Rückstand aus Isopropanol/Diisopropyläther (Volumenverhältnis 4 : 1) umkristallisiert.
Ausbeute: 6,72 g (80 % der Theorie)
$C_{12}H_{15}N_3O_2S$ (MG = 265,34); Schmelzpunkt 180° C
Analyse:

Ber.: C 54,32 % H 5,69 % N 15,83 % S 12,08 %
Gef.: C 54,41 % H 5,65 % N 15,75 % S 12,13 %

3) 3-Cyan-2,6-dimethyl-4-(4-thiomorpholinyl-S,S-dioxid)-pyridin-1-oxid-hydrochlorid:
3 g (0,012 Mol) der Verbindung des Beispiels 1b wurden in 10 ml 30-%-iges Wasserstoffperoxid und 20 ml Eisessig gelöst. Diese Mischung erhitzte man 3 Stunden auf 70° C, setzte nach dem Abkühlen auf Raumtemperatur 100 ml Isopropanol hinzu, rührte für weitere 30 Minuten, dampfte die Lösung unter vermindertem Druck zur Trockne ein, nahm in Wasser auf und neutralisierte die Lösung mit Natriumhydrogencarbonat. Die wäßrige Phase wurde gegen Dichlormethan ausgeschüttelt. Man kochte die abgetrennte Dichlormethanphase mit Aktivkohle auf. Die Lösung wurde zur Trockne eingeengt und mit äthanolischer HCl ins Hydrochlorid übergeführt. Man kristallisierte aus Isopropanol um. Ausbeute: 3,4 g (91 % der Theorie).
$C_{12}H_{16}ClN_3O_3S$ (MG 317,80); Schmelzpunkt 246° C
Analyse:

Ber.: C 45,36 % H 5,08 % Cl 11,15 % N 13,22 % S 11,08 %
Gef.: C 45,11 % H 5,19 % Cl 11,07 % N 13,01 % S 10,85 %

Die gleiche Verbindung ließ sich in analoger Weise auch aus der Verbindung gemäß Beispiel 2 erhalten.
4) 3-Cyan-2,6-dipropyl-4-(4-thiomorpholinyl)-pyridin-1-oxid-hydrochlorid:
14 g (0,066 Mol)3-Amino-4-chlor-2,6-dipropylpyridin wurden in einer Mischung aus 9,4 g konzentrierter Schwefelsäure und 70 ml Wasser gelöst. Man kühlte diese Lösung auf 0° C und tropfte eine Lösung von 4,8 g (0,07 Mol) Natriumnitrit in 14 ml Wasser dazu. Die so erhaltene Diazoniumsalzlösung ließ man in eine siedende Lösung von 5,9 g (0,07 Mol) Kupfer(I)cyanid und 12,92 g (0,198 Mol) Kaliumcyanid in 100 ml Wasser einfließen. Nach kurzem weiteren Erhitzen war die Reaktion beendet. Das Reaktionsgemisch wurde stark alkalisch gestellt und ausgeäthert. Die Ätherphase hinterließ nach dem Eindampfen 6 g (0,027 Mol) rohes 4-Chlor-3-cyan-2,6-dipropylpyridin, das in 100 ml Dichlormethan aufgenommen, mit 11,6 g (0,054 Mol) 80 %-iger m-Chlorperoxybenzoesäure versetzt und danach bei Raumtemperatur 12 Stunden gerührt wurde. Man filtrierte die Lösung, schüttelte gegen eine gesättigte Kaliumcarbonatlösung aus und engte die Dichlormethanphase ein. Die so erhaltenen 6 g (0,025 Mol) des Pyridin-1-oxids wurden, wie bei Beispiel 1 beschrieben, mit 5,18 g (0,05 Mol) Thiomorpholin in Isopropanol umgesetzt. Das erhaltene Rohprodukt ließ sich durch Säulenchromatographie an Kieselgel mit Essigester/Cyclohexan (Volumenverhältnis 4 : 1) als Fließmittel reinigen. Man erhielt ein gelb gefärbtes Öl, das nach längerem Stehen auskristallisierte. Ausbeute: 2,3 g (42 % der Theorie)
$C_{16}H_{24}ClN_3OS$ (MG: 341,91); Schmelzpunkt 34° C
Analyse:

Ber.: C 56,21 H 7,08 Cl 10,37 N 12,29 S 9,38
Gef.: C 56,01 H 7,19 Cl 10,41 N 12,06 S 9,37

5) 2,6-Dimethyl-4-(4-morpholinyl)-3-nitropyridin-1-oxid:
4 g (0,016 Mol) 2,6-Dimethyl-4-(4-morpholinyl)-3-nitropyridin wurden in 200 ml Dichlormethan gelöst und 3,8 g (0,018 Mol) 80 %-ige 3-Chlorperoxybenzoesäure zugefügt. Nach 20 Stunden filtrierte man den ausgefallenen Niederschlag ab, engte das Filtrat zur Trockne ein und schüttelte den Rückstand zwischen einer gesättigten Kaliumcarbonat-Lösung und Dichlormethan aus. Der Eindampfrückstand der organischen Phase wurde aus Äthanol/Diisopropyläther (Volumenverhältnis 3 : 1) umkristallisiert.
Ausbeute: 5,6 g (56 % der Theorie)
$C_{11}H_{16}ClN_3O_4$ (MG: 289,72); Schmelzpunkt 189° C

Analyse:

Ber.: C 45,60 H 5,57 Cl 12,23 H 14,50
Gef.: C 45,95 H 5,64 Cl 11,98 H 14,40

6) <u>2,6-Dimethyl-4-(4-thiomorpholinyl)-pyridin-3-carbonsäureamid-1-oxid:</u>
3 g (0,012 Mol) 3-Cyan-2,6-dimethyl-4-(4-thiomorpholinyl)-pyridin-1-oxid-hydrochlorid (aus Beispiel 1) wurden in 10 ml 10 N Natronlauge gelöst und 6 Stunden unter Rückfluß erhitzt. Danach engte man bis zur Bildung eines Niederschlages ein, kühlte, filtrierte den Feststoff ab und kristallisierte aus Methanol um. Ausbeute: 2,5 g (78 % der Theorie)
$C_{12}H_{17}N_3O_2S$ (MG: 267,35); Schmelzpunkt 285 - 287° C
Analyse:

Ber.: C 53,91 H 6,41 N 15,72 S 11,99
Gef.: C 53,93 H 6,44 N 15,64 S 12,10

7) <u>2,6-Dimethyl-4-(4-thiomorpholinyl)-pyridin-3-carbonsäure-1-oxid-hydrochlorid:</u>
38,2 g (0,129 Mol) 2,6-Dimethyl-4-thiomorpholinylpyridin-1-oxid-3-carbonsäureäthylester (s. Tabelle 1, Beispiel 19), 10,6 g (0,189 Mol) festes Kaliumhydroxid und 100 ml Äthylenglykol wurden 5 Stunden auf 170° C erhitzt. Man verdünnte anschließend mit Wasser, neutralisierte mit 6 N Salzsäure und destillierte das Wasser und Äthylenglykol unter vermindertem Druck ab. Der Rückstand wurde in Äthanol aufgenommen, durch Filtration vom unlöslichen Bestandteil befreit, mit Aktivkohle aufgekocht, nochmals filtriert und mit äthanolischer HCl versetzt. Man engte zur Trockne ein und kristallisierte das Hydrochlorid aus Methanol um. Ausbeute: 9,6 g (24,8 % der Theorie)
$C_{12}H_{17}ClN_2O_3S$ (MG: 304,79); Schmelzpunkt 222° C (Zers.)
Analyse:

Ber.: C 47,29 H 5,62 Cl 11,63 H 9,19 S 10,52
Gef.: C 47,56 H 5,63 Cl 11,23 H 9,22 S 10,40

Die vorgenannten und die auf analoge Weise hergestellten Verbindungen sind in nachstehender Tabelle 1 zusammengefaßt.

**Tabelle 1:** Verbindungen gemäß Formel I

| Beispiel | $R^1$ | $R^2$ | $-NR^3R^4$ | X | isoliert als | Schmelzpunkt ° C |
|---|---|---|---|---|---|---|
| 1 | $CH_3$ | $CH_3$ | 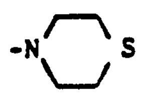 | CN | HCl | 237 - 239 |
| 2 | $CH_3$ | $CH_3$ | 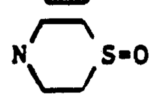 | CN | Base | 180 |
| 3 | $CH_3$ | $CH_3$ | 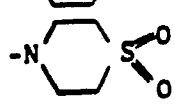 | CN | HCl | 246 |
| 4 | $C_3H_7$ | $C_3H_7$ | 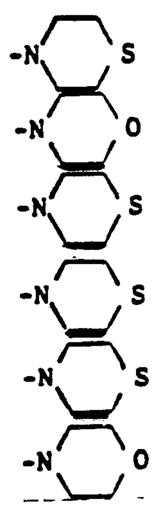 | CN | HCl | 34 |
| 5 | $CH_3$ | $CH_3$ | | $NO_2$ | HCl | 189 |
| 6 | $CH_3$ | $CH_3$ | | $CONH_2$ | Base | 285 - 287 |
| 7 | $CH_3$ | $CH_3$ | | COOH | HCl | 222 |
| 8 | $CH_3$ | $CH_3$ | | $NO_2$ | HCl | 204 - 206 |
| 9 | $CH_3$ | $CH_3$ | | CN | HCl | 245 |

| | | | | | | |
|---|---|---|---|---|---|---|
| 10 | $CH_3$ | $CH_3$ | 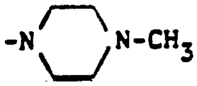 | CN | 2 HCl x $H_2O$ | 265 - 267 |
| 11 | $CH_3$ | $CH_3$ | 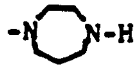 | $NO_2$ | 2 HCl | 240 |
| 12 | $CH_3$ | $CH_3$ | 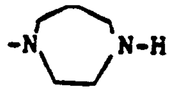 | CN | HCl | 218 - 220 |
| 13 | $CH_3$ | $CH_3$ | 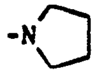 | CN | HCl | 209 |
| 14 | $CH_3$ | $CH_3$ | 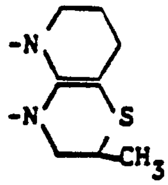 | CN | HCl | 223 |
| 15 | $CH_3$ | $CH_3$ | | CN | HCl | 211 |
| 16 | $CH_3$ | $CH_3$ | 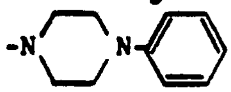 | CN | HCl | 195 |
| 17 | $CH_3$ | $CH_3$ | 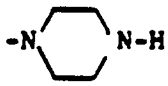 | CN | Base | 243 |
| 18 | $CH_3$ | $CH_3$ | 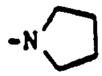 | $COOC_2H_5$ | Base | 151 |
| 19 | $CH_3$ | $CH_3$ | 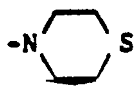 | $COOC_2H_5$ | Base | 98 |
| 20 | $CH_3$ | $CH_3$ | 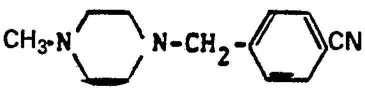 | CN | 2 HCl | 245 |
| 21 | $CH_3$ | $CH_3$ | 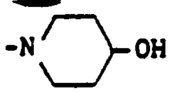 | CN | Base | 191 |
| 22 | $CH_3$ | $CH_3$ | 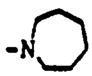 | CN | Base | 143 - 144 |
| 23 | $CH_3$ | $CH_3$ | 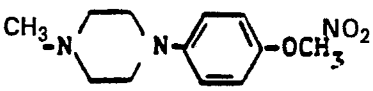 | $NO_2$ | 2 HCl | 170 |
| 24 | $CH_3$ | $CH_3$ | 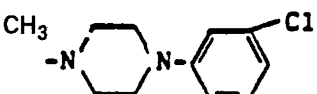 | $NO_2$ | 2 HCl | 176 |
| 25 | $CH_3$ | $CH_3$ | 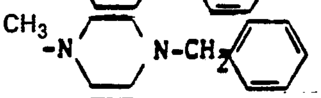 | $NO_2$ | 2 HCl x $H_2O$ | 208 |
| 26 | $CH_3$ | $CH_3$ | 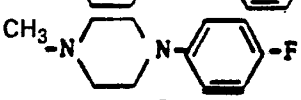 | $NO_2$ | 2 HCl | 196 |
| 27 | $CH_3$ | $CH_3$ | 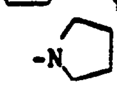 | $NO_2$ | HCl | 196 |
| 28 | $CH_3$ | $CH_3$ | 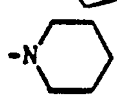 | $NO_2$ | HCl | 171 |
| 29 | $CH_3$ | $CH_3$ | $-NH-CH_2-CH_2-SH$ | CN | HCl | 212 |
| 30 | $CH_3$ | $C_6H_{13}$ | 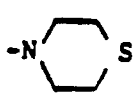 | CN | HCl x $H_2O$ | 104 |

**Pharmakologische Prüfung und Ergebnisse**

1. Antihypoxische Wirkung

Die Prüfung der erfindungsgemäßen Verbindungen auf gehirnprotektive Wirkung gegenüber Hypoxiebedingten Schädigungen erfolgte mit dem EEG-Hypoxietoleranztest (EEG: Elektroencephalogramm) im Vergleich mit den wichtigsten Vertretern der zum Stand der Technik gehörenden Verbindungstypen. Bei dieser Methode werden männliche, narkotisierte Ratten mit implantierten EEG-Elektroden einer progressiven Hypoxie durch Einblasen von Stickstoff in eine Kammer ausgesetzt. Dadurch kommt es nach etwa 2 Minuten zum Erlöschen der elektrischen Hirnfunktion, d. h. das EEG wird isoelektrisch. Unmittelbar nach Eintritt der Isoelektrizität öffnet man die Hypoxiekammer, worauf nach etwa 60 Sekunden bei unbehandelten Kontrolltieren wieder erste EEG-Signale registriert werden können. Als Beurteilungskriterium für eine antihypoxische Wirkung dient die EEG-Hypoxieerholungslatenz (EEG-EL), worunter die Zeitspanne vom Öffnen der Kammer nach Eintritt der EEG-Stille bis zum Wiederauftreten erster Signale zu verstehen ist. Eine protektiv, d. h. antihypoxisch, wirkende Substanz führt zu einer Verkürzung der EEG-EL im Vergleich mit einer unbehandelten Kontrollgruppe, wobei die prozentuale Änderung als Maß für die Aktivität dient. Die erfindungsgemäßen Verbindungen wurden 15 Minuten vor Beginn der Hypoxie intraperitoneal verabreicht. Die Gruppengröße betrug n = 8.

2. Akute Toxizität

Die Bestimmung der $LD_{50}$-Werte bzw. $LD_{50}$-Bereiche erfolgte standardgemäß über die innerhalb von 7 Tagen bei Naval Medical Research Institute-Mäusen nach einmaliger intravenöser (i. v.), intraperitonealer (i. p.) bzw. oraler (p. o.) Gabe auftretende Mortalität.

Die Ergebnisse dieser Untersuchungen, die die Überlegenheit der erfindungsgemäßen Verbindungen der Formel I gegenüber bekannten Vergleichspräparaten belegen, sind in Tabelle 2 zusammengefaßt.

**Tabelle 2:** Pharmakologische Testergebnisse

| Verbindung des Beispiels | EEG-Hypoxietoleranztest Dosis i. p. in mg/kg | Änderung der EEG-EL in % | Toxizität $LD_{50}$ (Maus) in mg/kg |
|---|---|---|---|
| 1 | 25 | - 50 | i. v.: 135<br>p. o.: 1190 |
| 5 | 25 | - 16 | i. v.: >200 |
| 11 | 50 | - 23 | i. v.: 200 |
| 13 | 50 | - 32 | i. v.: 200 |
| 14 | 50 | - 44 | i. v.: 100 - 200 |
| 22 | 25 | - 38 | i. v.: 50 - 100 |
| 26 | 25 | - 27 | i. v.: 200 |
| 28 | 25 | - 12 | i. v.: 100 - 200 |
| 3-Cyan-2,6-dimethyl-4-(4-thiomorpholinyl)-pyridin (Beispiel 13 aus DOS 2 900 504) | 50<br>25 | ± 0<br>± 0 | i. p.: 150 - 300 |
| 3-Phenoxypyridin (J. Med. Chem. 24, 346 - 250 (1981)) | 25 | - 14 | p. o.: 400 - 630 |
| 3-Cyan-2,6-dimethyl-4-(2-nitrophenyl)-pyridin-5-carbonsäuremethylester (DOS 3 209 274) | 50 | + 16 | i. v.: 50 - 100 |

Die gute gehirnprotektive Wirkung der erfindungsgemäßen Verbindungen ließ sich auch in weiteren Spezialversuchen eindrucksvoll bestätigen, wobei sich ebenfalls eine eindeutige Überlegenheit, diesmal gegenüber Piracetam (2-Oxo-1-pyrrolidinacetamid), einem häufig zur Therapie von Gehirnerkrankungen eingesetzten Mittel (S. Hoyer, Med. Prax. 79, 22 - 34 (1984)), ergab:

a) Einfluß auf die mit Scopolamin induzierte retrograde Amnesie im "passive avoidance"-Test

Die Testanordnung besteht aus einer Hell-Dunkel-Box mit elektrifizierbarem Gitterboden im dunklen Teil. 90 Minuten nach Verabreichung von Placebo und Präparat werden unerfahrene Mäuse männlichen Geschlechts mit Scopolamin-hydrobromid (3 mg/kg) subkutan behandelt. 5 Minuten später werden die Mäuse in den hellen Teil der Box gesetzt. Nach dem Überwechseln in den dunklen Teil erhalten sie dort einen als unangenehm empfundenen elektrischen Fußschock. 24 Stunden danach wird jede Maus einmal in den hellen Teil der Testapparatur gesetzt und die Verweildauer (max. 180 Sekunden) gemessen. Dabei zeigte sich bei den mit einer aktiven Dosis eines Präparates und Scopolamin behandelten Tieren ebenso wie bei den nicht mit

Scopolamin behandelten Tieren eine lange Verweildauer, während jene, die nur Placebo und Scopolamin erhalten haben, nur kurze Zeit verweilen. Die signifikante Wirkung einer Testsubstanz wird mittels Mediantest durch Vergleich mit der Kontrollgruppe berechnet. Als minimale effektive Dosis (MED) eines Präparates gilt jene, die eine signifikante Wirkung gegen Scopolamin hervorruft.

In diesem Test erwies sich beispielsweise die Verbindung aus Beispiel 1 bei oraler Verabreichung mit einer MED von 25 mg/kg viermal stärker wirksam als Piracetam, für das der entsprechende MED-Wert mit 100 mg/kg ermittelt wurde.

b) Gamma-Butyrolacton-Test an der Ratte

Aus der Literatur ist bekannt (L. I. Wolfson et al., J. Neurochem. 29, 777 (1977)), daß Gamma-Butyrolacton (GBL) u. a. Stoffwechselstörungen im Gehirn hervorruft, die sich anhand von Veränderungen im EEG verfolgen lassen. Die Verbindungen der Formel I vermögen diese GBL-Wirkung auf das EEG zu antagonisieren. So zeigte beispielsweise die Verbindung des Beispiels 1 nach intraperitonealer Verabreichung von 100 und 200 mg/kg eine starke, dosisabhängige Schutzwirkung, die jene des Vergleichspräparates Piracetam bei weitem übertraf.

**Patentansprüche**

1. Mehrfach substituierte Pyridin-1-oxide der Formel I

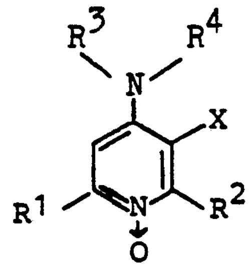

(I)

in der
$R^1$ und $R^2$ jeweils gleich oder verschieden sind und für Alkyl mit 1 bis 6 C-Atomen stehen,
$R^3$ Wasserstoff und
$R^4$ Mercaptoalkyl mit bis zu 4 C-Atomen bedeuten oder
$R^3$ und $R^4$ zusammen mit dem 4-ständigen N-Atom einen unsubstituierten oder bis zu zwei gleiche oder verschiedene Substituenten aus der Gruppe Hydroxy und Alkyl mit bis zu 2 C-Atomen tragenden fünf- bis siebengliedrigen gesättigten heterocyclischen Ring mit bis zu 2 Heteroatomen bilden, wobei das zweite Heteroatom Sauerstoff, Schwefel, der bis zu zwei Sauerstoffatome tragen kann, oder Stickstoff in Form der $NR^6$-Gruppe darstellt, Wasserstoff, Alkyl mit bis zu 2 C-Atomen, Phenylalkyl mit bis zu 2 C-Atomen im Alkylteil oder Phenyl ist und die Phenylringe der beiden letztgenannten Reste bis zu zwei gleiche oder verschiedene Substituenten aus der Gruppe Halogen und Methoxy tragen können, und
X die Cyan- oder Nitrogruppe oder die Gruppe $-CO-R^5$ bedeutet, in der $R^5$ für die Amino-, Hydroxy- oder eine Alkoxygruppe mit bis zu 4 C-Atomen steht,
und die physiologisch verträglichen Salze dieser Verbindungen.

2. Verbindungen und deren Salze nach Anspruch 1, gekennzeichnet durch wenigstens eines der Merkmale, daß $R^1$ und $R^2$ zusammen nicht mehr als 8 und insbesondere nicht mehr als 6 C-Atome enthalten, daß das Halogen am Phenylring Fluor, Chlor oder Brom ist und daß in $R^4$ der Mercaptoalkylrest bis zu 2 C-Atome enthält.

3. Verbindungen und deren Salze nach Anspruch 1 oder 2, dadurch gekennzeichnet, daß in Formel I
$R^1$ und $R^2$ jeweils 1 bis 3 C-Atome haben und insbesondere für Methyl stehen,
X die Cyan- oder Nitrogruppe bedeutet und die Gruppierung $NR^3R^4$ einen unsubstituierten oder mit einem Alkyl mit bis zu 2 C-Atomen substituierten heterocyclischen Ring mit mindestens 4 C-Atomen vorzugsweise einen Thiomorpholin-, Morpholin-, Pyrrolidin-, Piperidin-, Hexamethylenimin-, Piperazin- oder Homopiperazin-Ring oder einen unsubstituierten oder im Phenylkern mit einem Halogen substituierten 4-Phenylpiperazinrest darstellt.

4. Verbindungen und deren Salze nach Anspruch 3, dadurch gekennzeichnet, daß in Formel I
$R^1$ und $R^2$ jeweils Methyl,
X die Cyangruppe und
$NR^3R^4$ den Thiomorpholin-, Piperidin- oder Hexamethylenimin-Ring bedeuten.

5. Verbindung und deren Salze nach Anspruch 4, dadurch gekennzeichnet, daß es sich um 3-Cyan-2,6-dimethyl-4-(4-thiomorpholinyl)-pyridin-1-oxid handelt.

6. Verfahren zur Herstellung von mehrfach substituierten Pyridin-1-oxiden der Formel I gemäß einem oder mehreren der Ansprüche 1 bis 5 und ihren physiologisch verträglichen Salzen, dadurch gekennzeichnet, daß man

a) eine Verbindung der Formel II

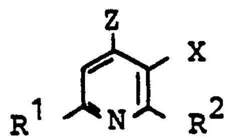

(II)

zu einem Pyridin-1-oxid der Formel III

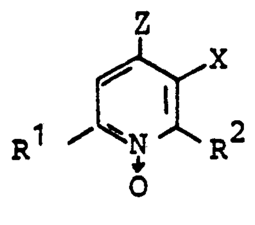

(III)

oxydiert und dieses anschließend mit einem Amin der Formel $HNR^3R^4$ (IV) zu einem Pyridin-1-oxid der Formel I umsetzt, wobei $R^1$ bis $R^4$ und X die in den Ansprüchen 1 bis 5 genannten Bedeutungen haben und Z ein Halogenatom darstellt, oder

b) eine Verbindung der Formel V,

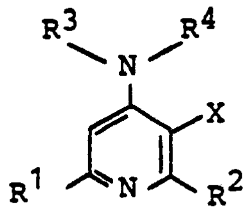

(V)

in der $R^1$ bis $R^4$ und X die vorgenannten Bedeutungen haben, zu einem Pyridin-1-oxid der Formel I oxydiert, wobei eine etwa in dem Rest $-NR^3R^4$ enthaltende Thioätherfunktion gleichzeitig mitoxydiert wird, und das gemäß a) oder b) erhaltene Produkt isoliert oder

c) zur Herstellung einer Verbindung der Formel I, in der die $NR^3R^4$-Gruppe eine Sulfoxy- oder Sulfongruppe enthält, eine nach dem Verfahren a) erhaltene Thioätherverbindung nachträglich, gegebenenfalls schrittweise, am Schwefel oxydiert oder

d) zur Herstellung einer Verbindung der Formel I, in der X für den Rest $-CONH_2$ steht, eine 3-Cyanverbindung der Formel I zum Amid hydrolysiert oder einen 3-Carbonsäureester der Formel I mit Ammoniak umsetzt oder

e) zur Herstellung einer Verbindung der Formel I, in der X die Carboxylgruppe bedeutet, einen 3-Carbonsäureester der Formel I hydrolysiert,

wobei man die Verbindungen der Formel I entweder in freier Form isoliert oder mit physiologisch verträglichen Säuren oder, für den Fall, daß X eine COOH-Gruppe darstellt, auch mit physiologisch verträglichen Basen physiologisch verträgliche Salze bildet.

7. Verfahren nach Anspruch 6, gekennzeichnet durch wenigstens eines der Merkmale, daß man die Oxydation von Verbindungen der Formeln II bzw. V zu den Pyridin-1-oxiden der Formeln III bzw. I mit Percarbonsäuren, vorzugsweise 3-Chlorperoxybenzoesäure, in einem Lösungs- oder Verteilungsmittel bei Temperaturen zwischen 10°C und dem Siedepunkt des Reaktionsmediums, vorzugsweise bei Raumtemperatur durchführt; daß man bei der Umsetzung von Verbindungen der Formel III mit den Aminen der Formel IV in einem Lösungs- oder Verteilungsmittel bei Temperaturen zwischen 0°C und dem Siedepunkt des Reaktionsmediums, vorzugsweise zwischen 20° und 100°C arbeitet; daß man die Oxydation von Verbindungen der Formel I mit einer Thioäthergruppe in dem $NR^3R^4$-Rest zu Sulfoxiden mit Perjodaten und zu Sulfonen mit Wasserstoffperoxid in Gegenwart von Essigsäure vornimmt; und daß man die Hydrolyse der 3-Cyanverbindungen der Formel I zu den entsprechenden Amiden und die Verseifung der 3-Carbonsäureester der Formel I zu den korrespondierenden Carbonsäuren unter alkalischen Bedingungen ausführt.

8. Arzneimittel, gekennzeichnet durch einen Gehalt an oder bestehend aus - a) mindestens einer Verbindung der Formel I oder b) mindestens einem ihrer physiologisch verträglichen Salze oder einer Kombination von a) und b) nach einem oder mehreren der Ansprüche 1 bis 5 oder mindestens einer nach dem Verfahren gemäß den Ansprüchen 6 oder 7 hergestellten Verbindung.

9. Arzneimittel nach Anspruch 8, dadurch gekennzeichnet, daß sie zur Vorbeugung und Behandlung von vaskulär und degenerativ bedingten Gehirnerkrankungen bestimmt sind und vorzugsweise in Form von festen Dosierungseinheiten mit einem Gehalt bis zu 1000, insbesondere von 100 bis 300 mg, oder in Form von

Injektionslösungen in Ampullen-Form mit einem Gehalt bis zu 200 mg, vorzugsweise 20 bis 100 mg vorliegen.

10. Verwendung von a) mehrfach substituierten Pyridin-1-oxiden der Formel I oder b) ihren physiologisch verträglichen Salzen oder c) einer Kombination von a) und b) zur Herstellung von Arzneimitteln, die zur Vorbeugung und Behandlung von vaskulär und degenerativ bedingten Gehirnerkrankungen bestimmt sind.

**Claims**

1. A multiply substituted pyridine 1-oxide of the formula I

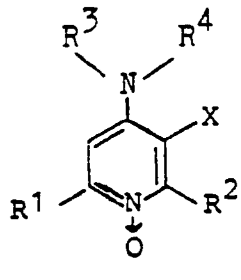

(I)

in which

$R^1$ and $R^2$ are, in each case, identical or different and represent alkyl having 1 to 6 carbon atoms,

$R^3$ denotes hydrogen, and

$R^4$ denotes mercaptoalkyl having up to 4 carbon atoms, or

$R^3$ and $R^4$ form, together with the nitrogen atom in the 4-position, a five- to seven-membered saturated heterocyclic ring which has up to 2 heteroatoms, the second heteroatom being oxygen, sulfur which can carry up to two oxygen atoms, or nitrogen in the form of the $NR^6$ group, in which $R^6$ is hydrogen, alkyl having up to 2 carbon atoms, phenylalkyl having up to 2 carbon atoms in the alkyl moiety or phenyl, and the phenyl rings in the two latter radicals can carry up to two identical or different substituents from the group, comprising halogen and methoxy, and which ring is unsubstituted or carries up to two identical or different substituents from the group comprising hydroxy and alkyl having up to 2 carbon atoms, and

X denotes the cyano or nitro group or the group $-CO-R^5$, in which $R^5$ represents the amino, hydroxyl or an alkoxy group having up to 4 carbon atoms, and the physiologically tolerated salts of this compound.

2. A compound and its salts as claimed in claim 1, which has at least one of the features that $R^1$ and $R^2$ together contain not more than 8 and, in particular, not more than 6, carbon atoms, that the halogen on the phenyl ring is fluorine, chlorine or bromine, and that the mercaptoalkyl radical in $R^4$ contains up to 2 carbon atoms.

3. A compound and its salts as claimed in claim 1 or 2, wherein, in formula I,

$R^1$ and $R^2$ each have 1 to 3 carbon atoms and represent, in particular, methyl,

X denotes the cyano or nitro group, and the group $NR^3R^4$ represents a heterocyclic ring which has at least 4 carbon atoms, preferably a thiomorpholine, morpholine, pyrrolidine, piperidine, hexamethyleneimine, piperazine or homopiperazine ring, and which is unsubstituted or substituted by one alkyl having up to 2 carbon atoms, or represents a 4-phenylpiperazine radical which is unsubstituted or substituted in the phenyl nucleus by one halogen.

4. A compound and its salts as claimed in claim 3, wherein, in formula I,

$R^1$ and $R^2$ each denote methyl,

X denotes the cyano group, and

$NR^3R^4$ denotes the thiomorpholine, piperidine or hexamethyleneimine ring.

5. A compound and its salts as claimed in claim 4, which is 3-cyan-2,6-dimethyl-4-(4-thiomorpholinyl)pyridine 1-oxide.

6. A process for the preparation of multiply substituted pyridine 1-oxides of the formula I as claimed in one or more of claims 1 to 5, and of their physiologically tolerated salts, which comprises

a) oxidation of a compound of the formula II

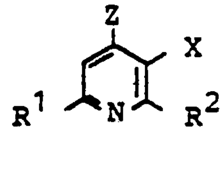

(II)

to a pyridine 1-oxide of the formula III

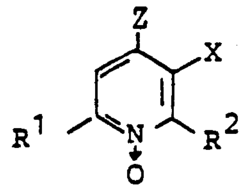

(III),

and then reaction of the latter with an amine of the formula $HNR^3R^4$ (IV) to give a pyridine 1-oxide of the formula I, $R^1$ to $R^4$ and X having the meanings mentioned in claims 1 - 5, and Z representing a halogen atom,

b) oxidation of a compound of the formula V,

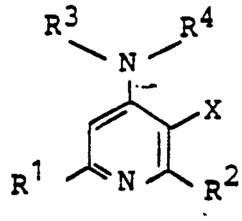

(V)

in which $R^1$ to $R^4$ and X have the abovementioned meanings, to a pyridine 1-oxide of the formula I, any thioether group contained in the radical $-NR^3R^4$ simultaneously also being oxidized,

and isolation of the product obtained according to a) or

b) or

c) for the preparation of a compound of the formula I in which the $NR^3R^4$ group contains a sulfoxy or sulfone group, subsequent oxidation, where appropriate stepwise, of the sulfur in the thioether compound obtained by process a), or

d) for the preparation of a compound of the formula I in which X represents the radical $-CONH_2$, hydrolysis of a 3-cyano compound of the formula I to the amide, or reaction of a 3-carboxylic ester of the formula I with ammonia, or

e) for the preparation of a compound of the formula I in which X denotes the carboxyl group, hydrolysis of a 3-carboxylic ester of the formula I,

the compounds of the formula I being either isolated in the free form or forming physiologically tolerated salts with physiologically tolerated acids or, in the case where X represents a COOH group, with physiologically tolerated bases.

7. The process as claimed in claim 6, which has at least one of the features that the oxidation of compounds of the formulae II and V to give the pyridine 1-oxides of the formulae III and I respectively is carried out with percarboxylic acids, preferably 3-chloroperoxybenzoic acid, in a solvent or dispersant at temperatures between 10°C and the boiling point of the reaction medium, preferably at room temperature; that the reaction of compounds of the formula III with the amines of the formula IV is carried out in a solvent or dispersant at temperatures between 0°C and the boiling point of the reaction medium, preferably between 20° and 100°C; that the oxidation of compounds of the formula I with a thioether group in the $NR^3R^4$ radical is effected with periodates to give sulfoxides and with hydrogen peroxide in the presence of acetic acid to give sulfones; and that the hydrolysis of the 3-cyano compounds of the formula I to give the corresponding amides, and the hydrolysis of the 3-carboxylic esters of the formula I to give the corresponding carboxylic acids, is carried out under alkaline conditions.

8. A medicament which contains or is composed of a) at least one compound of the formula I, or b) at least one of its physiologically tolerated salts, or a combination of a) and b) as claimed in one or more of claims 1 to 5, or at least one compound prepared by the process as claimed in claims 6 or 7.

9. A medicament as claimed in claim 8, which is intended for the prevention and treatment of brain disorders caused by vascular and degenerative factors, and is preferably in the form of solid dosage units containing up to 1000, in particular from 100 to 300, mg, or in the form of injection solutions in ampoule form containing up to 200 mg, preferably 20 to 100 mg.

10. The use of a) multiply substituted pyridine 1-oxides of the formula I, or b) their physiologically tolerated salts, or c) a combination of a) and b), for the preparation of medicaments which are intended for the prevention and treatment of brain disorders caused by vascular and degenerative factors.

**Revendications**

1. Oxydes-1 de pyridine plusieurs fois substitués, de formule I

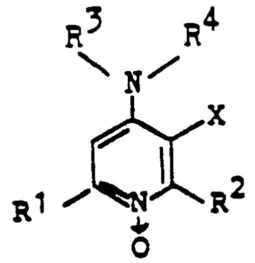

(I)

dans laquelle

$R^1$ et $R^2$ sont identiques ou différents, et représentent chacun un groupe alkyle ayant de 1 à 6 atomes de carbone,

$R^3$ représente un atome d'hydrogène et

$R^4$ représente un groupe mercaptoalkyle ayant jusqu'à 4 atomes de carbone, ou

$R^3$ et $R^4$ forment conjointement avec l'atome d'azote en position 4 un hétérocycle saturé ayant de 5 à 7 chaînons, non substitué ou portant jusqu'à deux substituants identiques ou différents, choisis parmi le groupe hydroxy et des groupes alkyle ayant jusqu'à 2 atomes de carbone, hétérocycle comportant jusqu'à deux hétéroatomes, le second hétéroatome représentant un atome d'oxygène, de soufre pouvant porter jusqu'à deux atomes d'oxygène, ou d'azote sous forme du groupe $NR^6$, dans lequel $R^6$ est un atome d'hydrogène ou un groupe alkyle ayant jusqu'à 2 atomes de carbone, phénylalkyle ayant jusqu'à 2 atomes de carbone dans le fragment alkyle, ou phényle, et les noyaux phényle des deux radicaux cités en dernier pouvant porter jusqu'à deux substituants identiques ou différents, choisis parmi des halogènes et le groupe méthoxy, et

X représente le groupe cyano ou nitro, ou un radical $-CO-R^5$ dans lequel $R^5$ représente le groupe amino, hydroxy ou un groupe alcoxy ayant de 1 à 4 atomes de carbone,

et sels physiologiquement acceptables de ces composés.

2. Composés et leurs sels selon la revendication 1, caractérisés par au moins une des caractéristiques que $R^1$ et $R^2$ ne contiennent ensemble pas plus de 8, et en particulier pas plus de 6 atomes de carbone, que l'halogène sur le noyau phényle est le fluor, le chlore ou le brome, et que le groupe mercaptoalkyle dans $R^4$ contient jusqu'à 2 atomes de carbone.

3. Composés et leurs sels selon la revendication 1 ou 2, caractérisés en ce que, dans la formule I,

$R^1$ et $R^2$ ont chacun de 1 à 3 atomes de carbone et représentent en particulier le groupe méthyle,

X représente le groupe cyano ou nitro, et le groupement

$NR^3R^4$ représente un hétérocycle ayant au moins 4 atomes de carbone, non substitué ou substitué par un groupe alkyle ayant jusqu'à 2 atomes de carbone, de préférence un cycle thiomorpholine, morpholine, pyrrolidine, pipéridine, hexaméthylène-imine, pipérazine ou homopipérazine, ou un radical 4-phénylpipérazine non substitué ou substitué par un halogène sur le noyau phényle.

4. Composés et leurs sels selon la revendication 3, caractérisés en ce que, dans la formule I,

$R^1$ et $R^2$ représentent chacun le groupe mêthyle,

X représente le groupe cyano et

$NR^3R^4$ représente le cycle thiomorpholine, pipéridine ou hexaméthylène-imine.

5. Composés et leurs sels selon la revendication 4, caractérisés en ce qu'il s'agit de l'oxyde-1 de 3-cyano-2,6-diméthyl-4-(4-thiomorpholine)-pyridine.

6. Procédé pour la préparation d'oxydes-1 de pyridine plusieurs fois substitués, de formule 1, selon une ou plusieurs des revendications 1 à 5, et de leurs sels physiologiquement acceptables, caractérisé en ce que

a) on oxyde un composé de formule II

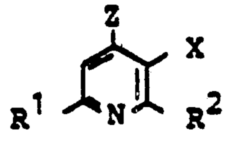

(II)

en un oxyde-1 de pyridine de formule III

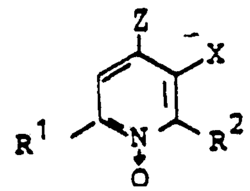

(III)

et on fait ensuite réagir celui-ci avec une amine de formule $HNR^3R^4$ (IV) pour aboutir à un oxyde-1 de pyridine de formule (I), $R^1$ à $R^4$ et X ayant les significations données dans les revendications 1 à 5, et Z représentant un atome d'halogène, ou

b) on oxyde un composé de formule V

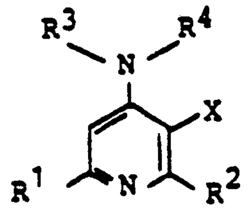

(V)

dans laquelle $R^1$ à $R^4$ et X ont les significations données plus haut, pour aboutir à un oxyde-1 de pyridine de formule I, une fonction thioéther contenue par exemple dans le reste $-NR^3R^4$ étant simultanément oxydée,

et on isole le produit obtenu selon a) ou b), ou

c) pour la préparation d'un composé de formule I dans lequel le reste $NR^3R^4$ contient un groupe sulfoxy ou sulfone, on oxyde ultérieurement sur le soufre, éventuellement progressivement, un composé thioéther obtenu selon le procédé a), ou

d) pour la préparation d'un composé de formule I dans lequel X représente le radical $-CONH_2$, on hydrolyse en l'amide un composé 3-cyano de formule I, ou on fait réagir un ester d'acide 3-carboxylique de formule I avec de l'ammoniac, ou

e) pour la préparation d'un composé de formule I dans lequel X représente le groupe carboxy, on hydrolyse un ester d'acide 3-carboxylique de formule I,

les composés de formule I étant soit isolés sous forme libre, soit mis sous forme de sels physiologiquement acceptables avec des acides physiologiquement acceptables, ou, dans le cas où X représente le groupe COOH, également avec des bases physiologiquement acceptables.

7. Procédé selon la revendication 6, caractérisé par au moins une des caractéristiques que l'on effectue l'oxydadation de composés de formules II ou V en les oxydes-1 de pyridine de formule III ou I, avec des peracides carboxyliques, de préférence l'acide 3-chloroperoxybenzoïque, dans un solvant ou dispersant, à des températures comprises entre 10°C et le point d'ébullition du milieu réactionnel, de préférence à la température ambiante; que dans la réaction de composés de formule III avec les amines de formule IV, on opère dans un solvant ou dispersant à des températures comprises entre 0°C et le point d'ébullition du milieu réactionnel, de préférence entre 20°C et 100°C; que l'on effectue l'oxydation en sulfoxydes de composés de formule I comportant un groupe thioéther dans le radical $NR^3R^4$, avec des periodates, et en sulfones avec le peroxyde d'hydrogène, en présence d'acide acétique; et que l'on effectue dans des conditions alcalines l'hydrolyse des composés 3-cyano de formule I en les amides correspondants et la saponification des esters d'acides 3-carboxyliques de formule I en les acides carboxyliques correspondants.

8. Médicaments caractérisés par une teneur en ou consistant en a) au moins un composé de formule I, ou b) au moins un de ses sels physiologiquement acceptables, ou en une combinaison de a) et b) selon une ou plusieurs des revendications 1 à 5, ou au moins en un composé préparé par le procédé selon la revendication 6 ou 7.

9. Médicaments selon la revendication 8, caractérisés en ce qu'ils sont conçus pour la prévention et le traitement d'affections cérébrales d'origine vasculaire et dégénérative et se trouvent de préférence sous forme de doses unitaires solides ayant une teneur allant jusqu'à 1 000, en particulier de 100 à 300 mg, ou sous forme de solutions injectables présentées en ampoule ayant une teneur allant jusqu'à 200 mg, de préférence de 20 à 100 mg.

10. Utilisation a) d'oxydes-1 de pyridine plusieurs fois substitués, de formule I, ou b) de leurs sels physiologiquement acceptables, ou c) d'une combinaison de a) et b), pour la préparation de médicaments qui sont conçus pour la prévention et le traitement d'affections cérébrales d'origine vasculaire et dégénérative.